# EUROPÄISCHE PATENTSCHRIFT

(11) **EP 1 252 159 B1**
(45) Veröffentlichungstag und Bekanntmachung des Hinweises auf die Patenterteilung: **21.04.2004**
(21) Anmeldenummer: 01900395.3
(22) Anmeldetag: 08.01.2001
(51) Int. Cl.: C07D 417/06

(54) **VERFAHREN ZUR HERSTELLUNG VON HETEROCYCLISCHEN VERBINDUNGEN**
METHOD FOR PRODUCING HETEROCYCLIC COMPOUNDS
PROCEDE DE PRODUCTION DE COMPOSES HETEROCYCLIQUES

(30) Priorität: 19.01.2000 DE 10002049
(43) Veröffentlichungstag der Anmeldung: 30.10.2002
(73) Patentinhaber: Bayer CropScience AG, 40789 Monheim (DE)
(72) Erfinder: SEIFERT, Hermann, 51467 Bergisch Gladbach (DE); STELZER, Uwe, 51399 Burscheid (DE); HEYN, Dr. Armin, 51467 Bergisch Gladbach (DE)
(86) Internationale Anmeldenummer: PCT/EP2001/000125
(87) Internationale Veröffentlichungsnummer: WO 2001/053296

(56) Entgegenhaltungen:
- EP-A- 0 163 855
- EP-A- 0 235 725
- EP-A- 0 260 485
- EP-A- 1 024 140

## Beschreibung

Die vorliegende Erfindung betrifft ein neues Verfahren zur Herstellung von bekannten heterocyclischen Verbindungen.

Bekannt ist die Herstellung ungesättigter, heterocyclischer Verbindungen durch die Alkylierung von unsubstituierten Ringstoffatomen, die unter anderem in Alkohol durchgeführt werden können (EP-A-259 738).

Ebenfalls bekannt sind Alkylierungsreaktionen in aprotischen Lösungsmitteln (EP-A-259 738, EP-A-235 725).

In diesen Fällen ist eine anschließende Reinigung des Produkts notwendig, um eine ausreichende Reinheit zu erreichen, zudem sind die mit den bekannten Verfahren zu erzielenden Ausbeuten unbefriedigend.

Es wurde gefunden, dass man Verbindungen der Formel (I) 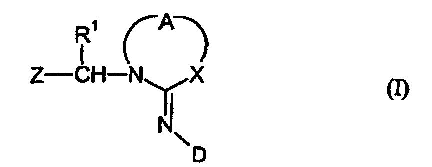 in welcher
- R¹: für ein Wasserstoffatom steht,
- A: für eine Ethylengruppe, die durch Alkyl substituiert sein kann, oder eine Trimethylengruppe, die durch Alkyl substituiert sein kann, steht,
- D: für Nitro oder Cyano steht,
- X: für ein Sauerstoff- oder Schwefelatom oder die Gruppen 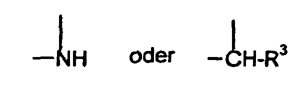 steht,
in welcher
R³ für ein Wasserstoffatom oder eine Alkylgruppe steht, und
- Z: für 2-Chlor-pyrid-5-yl steht,
erhält, indem man Verbindungen der Formel (II) 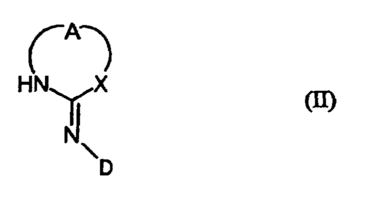 in der
A, D und X die oben angegebenen Bedeutungen haben,
mit einer Base und in Gegenwart eines Verdünnungsmittels umsetzt und das Reaktionsgemisch anschließend mit dem Gemisch aus CCMP/CMP (2-Chlor-5-chlormethylpyridin/2-Chlor-5-methylpyridin) und den entsprechenden Hydrochloriden umsetzt.

Überraschenderweise können nach dem erfindungsgemäßen Verfahren die o.g. Verbindungen auf einfachere, in einer geringeren Anzahl von Verfahrensschritten und in besserer Ausbeute, hergestellt werden.

In den allgemeinen Formeln (I) und (II) stehen die Variablen
- A: bevorzugt für eine Ethylen- oder Trimethylengruppe, die jeweils durch eine C₁-C₃-Alkylgrappe substituiert sein können, besonders bevorzugt für eine Ethylengruppe;
- D: für Nitro oder Cyano,
- X: bevorzugt für ein Sauerstoff- oder Schwefelatom oder für die Gruppe 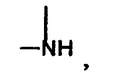 besonders bevorzugt für ein Sauerstoffatom oder die Gruppe

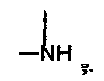

Eine ganz besonders bevorzugte Verbindung der Formel (I) ist die Verbindung der Formel (Ia) 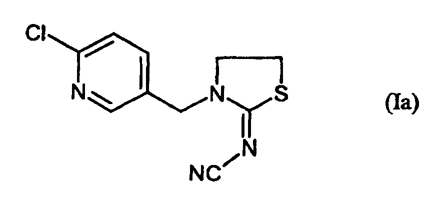 die durch Umsetzung der Verbindung der Formel (IIa) 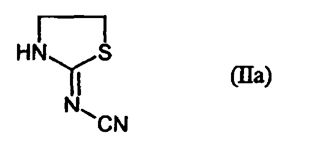 mit einer Base und in Gegenwart eines Verdünnungsmittels und durch anschließende Umsetzung des Reaktionsgemisches mit einem Gemisch aus CCMP/CMP mit den entsprechenden Hydrochloriden erhalten wird.

Eine weitere ganz besonders bevorzugte Verbindung der Formel (I) ist die Verbindung der Formel (Ib) 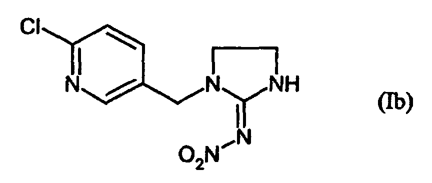 die durch Umsetzung der Verbindung der Formel (IIb) 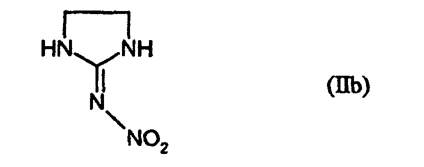 mit einer Base und in Gegenwart eines Verdünnungsmittels und durch anschließende Umsetzung des Reaktionsgemisches mit einem Gemisch aus CCMP/CMP mit den entsprechenden Hydrochloriden erhalten wird.

Als Lösungsmittel können protische und dipolar-aprotische Lösungsmittel, insbesondere Wasser, Alkohole, Ketone (vorzugsweise MIBK), Ester (vorzugsweise Essigsäurebutylester), Nitrile (vorzugsweise Acetonitril, n-Propionitril, Butyronitril), Pyridine (vorzugsweise CMP), Amide (DMF), DMSO oder Carbonate eingesetzt werden oder deren Gemische mit Wasser. Beim Einsatz von Alkoholen als Lösungsmittel können die Verbindungen der Formel (I) direkt in einer für den Einsatz als Pflanzenschutzmittel vorteilhaften Modifikation und der notwendigen Reinheit erhalten werden.

Als beispielhaft verwendete Alkohole seien genannt:
- primäre Alkohole wie Methanol, Ethanol, Propanol, Butanol, 2-Methyl-1-propanol, 1-Pentanol, Benzylalkohol,
- sekundäre Alkohole wie Isopropanol, sec.-Butanol, 2-Pentanol, tert.-Alkohole wie tert.-Butanol.

Als Lösungsmittel besonders bevorzugt sind mit Wasser nicht oder nur teilweise mischbare Alkohole, wie n-Butanol, Amylalkohol, insbesondere n-Butanol oder Nitrile, wie n-Propionitril oder Butyronitril, insbesondere n-Propionitril.

Das Verfahren wird in Gegenwart einer Base durchgeführt. Als Beispiele seien genannt: Alkali und Erdalkalihydroxide wie NaOH, KOH, Ca(OH)₂, Alkalimetallcarbonate oder Hydrogencarbonate wie Na₂CO₃, Li₂CO₃, K₂CO₃, Cs₂CO₃ oder NaHCO₃ und KHCO₃. Bevorzugt seien genannt K₂CO₃, NaOH und KHCO₃, insbesondere K₂CO₃.

Die Verbindungen der allgemeinen Formel (II) können auch als Alkali- oder Erdalkalisalz in fester oder gelöster Form eingesetzt werden.

Beim Arbeiten in Wasser, Wasser-Alkohol- oder Wasser-Nitril-Gemischen wird das Verfahren bei einem pH-Bereich zwischen 6 und 13 durchgeführt.

Als Katalysatoren können Phasentransferkatalysatoren, gegebenenfalls quartäre Ammoniumhalogenide wie Tetrabutylammoniumbromid oder -chlorid oder Cs-Salze etc. eingesetzt werden.

Die Reaktion kann auch durchgeführt werden, indem man die Verbindungen der allgemeinen Formel (II) gegebenenfalls als Alkali oder Erdalkalisalz vorlegt und in Gegenwart einer Base bei Temperaturen von 40°C bis 130°C, gegebenenfalls im Vakuum, vorzugsweise bei 100 bis 500 mbar erhitzt und anschließend das CCMP/CMP-Gemisch bei 50 bis 90°C, gegebenenfalls im Vakuum, vorzugsweise bei 60°C bis 80°C zudosiert.

Die Reaktion wird zweckmäßigerweise unter Atmosphärendruck durchgeführt, jedoch kann auch unter vermindertem oder erhöhtem Druck gearbeitet werden. Besonders bevorzugt ist die Durchführung im Vakuum.

Bei der praktischen Durchführung des Verfahrens wird beispielsweise 1 Mol eines Gemisches aus CCMP/CMP mit 0,95 bis 3 Mol der Verbindungen der Formel (II), vorzugsweise 1,0 bis etwa 2,5 Mol in einem Lösungsmittel wie Butanol in Anwesenheit von 1 bis 3 Mol, vorzugsweise 1,5 bis 2,5 Mol einer Base wie z.B. Kaliumcarbonat und gegebenenfalls in Gegenwart eines Katlaysators wie Tetrabutylammoniumbromid oder Cäsiumcarbonat umgesetzt.

Bei Verwendung von Wasser in einem Zweiphasensystem arbeitet man vorzugsweise bei pH 8-10.

Die Reaktionszeit liegt zwischen 3 und 12 Stunden, bevorzugt bei 5 bis 10 Stunden. Nach beendeter Reaktion kann gegebenenfalls ein Lösungsmittelwechsel erfolgen. Man destilliert hierbei den größten Teil des Reaktionsverdünnungsmittel im Vakuum (1-1000 mbar) ab und ergänzt die Menge durch eines der o.g. Verdünnungsmittel. Die Substitution des Lösungsmittels kann vor oder nach der Hydrolyse erfolgen.

Die Suspension aus der Reaktion wird bei einer Temperatur von 50°C bis 100°C hydrolysiert und es erfolgt die Trennung der organischen Phase bei 50°C bis 80°C. Diese wird abgekühlt, der ausgefallene Wirkstoff isoliert, gewaschen und umkristallisiert.

Das in der Mutterlauge befindliche CMP (Temperaturbereich 50°C bis 130°C, Druckbereich 1-1000 mbar) kann wiedergewonnen und in den Prozeß zurückgeführt werden: Die erhaltene Mutterlauge kann mit dem Verdünnungsmittel der Kristallisation versetzt werden (1 Teil Mutterlauge / 4 Teile Lösungsmittel - 1 Teil Mutterlauge / 0.5 Teile Lösungsmittel), die Suspension wird abgekühlt und der nachgefallene Wirkstoff abfiltriert.

Die Ausgangsstoffe der Formel (II) sind bekannt und/oder können nach an sich bekannten Verfahren hergestellt werden (vgl. JACS 79, (1957), 3565; Arch. Pharm. 305, (1972), 731-736; Heterocycles 31 (1990), 1601-1604; Biosci. Biotechnol. Biochem. 57, (1993),127-128; Biosci. Biotechnol. Biochem. 56, (1992), 364-365).

Die Darstellung von 2-Chlor-5-chlormethylpyridin gelingt analog dem beschriebenen Verfahren (EP-A-458 109, EP-A-260 485). Das 2-Chlor-5-methylpyridin wird in einem organischen Lösungsmittel (Acetonitril, Tetrachlorkohlenstoff, Wasser pHkontrolliert) mit Radikalstarter (AIBN) in der Siedehitze chloriert. Der Umsatz der Reaktion wird bei ca. 40 % abgebrochen, um eine hohe Selektivität an 2-Chlor-5-chlormethylpyridin zu erhalten. Anschließend erfolgt die Destillation des organischen Lösungsmittels im Vakuum.

Das Gemisch aus CCMP/CMP enthält nach der Destillation des Lösungsmittels 5-15% Restlösungsmittel, 30-50% CMP und 25-45 % CCMP mit den entsprechenden Hydrochloriden.

Dieses Gemisch aus 2-Chlor-5-methylpyridin und 2-Chlor-5-chlormethylpyridin und den entsprechenden Hydrochloriden dient als Einsatzstoff für die Wirkstoffreaktion. Dieses Gemisch kann unverdünnt oder in einem Verdünnungsmittel, welches zweckmäßigerweise auch in der Wirkstoffreaktion zum Einsatz kommt in diese eingesetzt werden.

Die Verbindungen der Formel (I) sind beispielsweise zur Verwendung als Insektizide geeignet (EP A2 0235 752, EP A2 0259 738).

Die folgenden Beispiele erläutern den Gegenstand der Erfindung ohne sie in irgend einer Weise einzuschränken.

### Beispiel 1

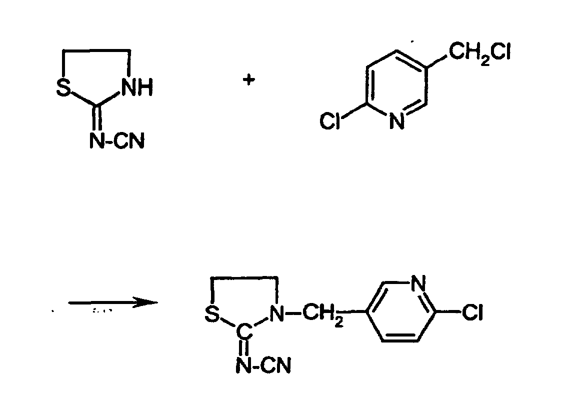

0,615 Mol Kaliumcarbonat und 0,3 Mol 2-Cyaniminothiazolidin werden in 100 ml n-Butanol suspendiert und bei 60°C 1 h gerührt. Innerhalb 2 h werden bei 70°C 0,315 Mol 2-Chlor-5-chlormethylpyridin/2-Chlor-5-methylpyridin (CCMP/CMP, 23 % CCMP im Gemisch) in 100 ml n-Butanol suspendiert zugegeben und 2 h bei 72°C gerührt. Nach Abkühlen auf 65°C werden 400 g Wasser zugegeben und die Phasen getrennt. Anschließend wird die organische Phase 3 h bei 50°C gerührt und anschließend wird 18 h bei -5°C gerührt. Ausgefallenes Produkt wird abfiltriert und getrocknet; 59,6 g (78 % der Theorie).

### Beispiel 2

0,615 Mol Kaliumcarbonat und 0,3 Mol 2-Cyaniminothiazolidin werden in 100 ml n-Butanol suspendiert und bei 60°C 1 h gerührt. Innerhalb 2 h werden bei 70°C 0,315 Mol 2-Chlor-5-chlormethylpyridin/2-Chlor-5-methylpyridin (CCMP/CMP, 23 % CCMP im Gemisch) in 100 ml n-Butanol suspendiert zugegeben und 2 h bei 72°C gerührt. Nach Abkühlen auf 65°C werden 400 g Wasser zugegeben und die Phasen getrennt. Anschließend wird die organische Phase 3 h bei 50°C gerührt und anschließend wird 18 h bei -5°C gerührt. Ausgefallenes Produkt wird abfiltriert und getrocknet. Die Mutterlauge wird im Verhältnis 1:1 mit Butanol versetzt und auf 0°C abgekühlt, der dabei ausfallende Feststoff wird abfiltriert und getrocknet. Gesamtausbeute: 66,1 g (85 % isoliertes Produkt).

### Beispiel 3

0,3 Mol 2-Cyaniminothiazolidin und 4,2 g Tetrabutylammoniumbromid werden in 300 ml Wasser suspendiert und auf 70°C erwärmt. Die Zugabe von 0,315 Mol CMP/CCMP-Gemisch erfolgt. Mit NaOH wird der pH-Wert des Reaktionsgemischs kontinuierlich auf 8 bis 8,5 gehalten. Nach 2 h Reaktionszeit bei 60°C wird bei dieser Temperatur eine Phasentrennung durchgeführt und die organische Phase mit 150 ml Butanol verdünnt und gerührt. Innerhalb von 3 h wird auf 3°C abgekühlt und ausgefallenes Produkt abgesaugt; 58,5 g (76 % der Theorie) werden so erhalten.

### Beispiel 4

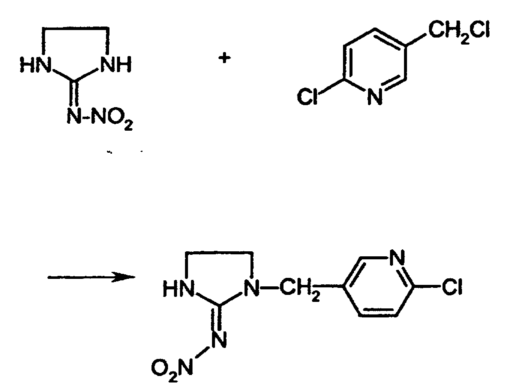

1,5 Mol Ethylennitroguanidin (enthält 16.5 % H₂O) werden in 600 g n-Propionitril aufgenommen und das Wasser azeotrop abdestilliert. Danach erfolgt die Zugabe von 342 g Kaliumcarbonat (2,5 Mol) bei 95°C. Anschließend gibt man 2 g Cäsiumcarbonat zu. Innerhalb von 30 Minuten werden 521 g CMP/CCMP (31 % CCMP) bei 95-100°C zugegeben. Nach 5 h Reaktionszeit bei 100-105°C werden 1,21 Wasser zugefügt. Mit HCl wird der pH-Wert des Reaktionsgemisches auf 6 bis 7 gehalten. Aus der organischen Phase wird das Propionitril bei 180 mbar abdestilliert. Danach gibt man 500 g n-Butanol zu und erhitzt auf 80°C und trennt die Phasen. Die organische Phase wird auf 0°C abgekühlt. Ausgefallenes Produkt wird abfiltriert und getrocknet; 187,4 g (73 % der Theorie).

## Patentansprüche

1. Verfahren zu Herstellung von Verbindungen der Formel (I) 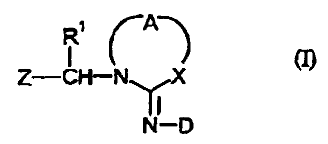 in welcher
R¹ für ein Wasserstoffatom steht,
A für eine Ethylengruppe, die durch Alkyl substituiert sein kann, oder eine Trimethylengruppe, die durch Alkyl substituiert sein kann, steht,
D für Nitro oder Cyano steht,
X für ein Sauerstoff- oder Schwefelatom oder die Gruppen 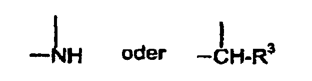 steht,
in welcher
R³ für ein Wasserstoffatom oder eine Alkylgruppe steht, und
Z für 2-Chlor-pyrid-5-yl steht,
**dadurch gekennzeichnet, dass** man Verbindungen der Formel (II) 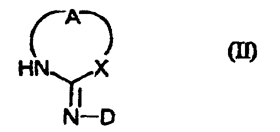 in der A, D und X die oben angegebenen Bedeutungen haben,
mit einer Base und in Gegenwart eines Verdünnungsmittels umsetzt und das Reaktionsgemisch anschließend mit dem Gemisch aus CCMP/CMP (2-Chlor-5-chlormethylpyridin/2-Chlor-5-methylpyridin) und den entsprechenden Hydrochloriden umsetzt.

2. Verfahren gemäß Anspruch 1, **dadurch gekennzeichnet, dass** die Reaktion in Gegenwart eines mit Wasser nicht oder nur teilweise mischbaren Alkohols oder Nitrils als Verdünnungsmittel durchgeführt wird.

3. Verfahren gemäß den Ansprüchen 1 oder 2, **dadurch gekennzeichnet, dass** das Verdünnungsmittel aus der Reihe n-Butanol, Amylalkohol, n-Propionitril oder Butyronitril ausgewählt ist.

4. Verfahren gemäß einem oder mehreren der Ansprüche 1 bis 3, **dadurch gekennzeichnet, dass** das Gemisch aus CCMP/CMP und den entsprechenden Hydrochloriden erhalten wird, indem man CMP in einem organischen Lösungsmittel mit Radikalstarter in der Siedehitze bis zu einem Umsatz von ca. 40 % chloriert und anschließend das organische Lösungsmittel im Vakuum destilliert.

5. Verfahren gemäß Anspruch 4, **dadurch gekennzeichnet, dass** das Gemisch aus CCMP/CMP und den entsprechenden Hydrochloriden nach der Destillation des Lösungsmittels 5-15 % Restlösungsmittel, 30-50 % CMP und 25-45 % CCMP mit den entsprechenden Hydrochloriden enthält.

6. Verfahren gemäß einem der Ansprüche 1 bis 5, **dadurch gekennzeichnet, dass** man als Verbindungen der Formel (II) die Verbindung der Formel (IIa) oder die Verbindung der Formel (IIb) 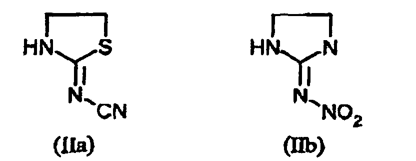 einsetzt.

## Claims

1. Process for the preparation of compounds of the formula (I) 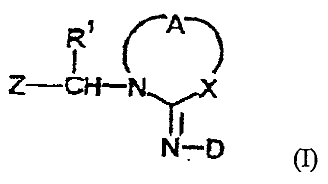 in which
R¹ is a hydrogen atom,
A is an ethylene group which may be substituted by alkyl, or a trimethylene group which may be substituted by alkyl,
D is nitro or cyano,
X is an oxygen or sulphur atom or the groups 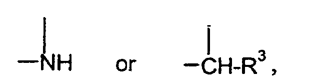 in which
R³ is a hydrogen atom or an alkyl group, and
Z is 2-chloropyrid-5-yl,
**characterized in that** compounds of the formula (II) 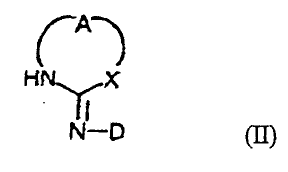 in which
A, D and X have the meanings given above,
are reacted with a base and in the presence of a diluent, and the reaction mixture is then reacted with the mixture of CCMP/CMP (2-chloro-5-chloromethylpyridine/2-chloro-5-methylpyridine) and the corresponding hydrochlorides.

2. Process according to Claim 1, **characterized in that** the reaction is carried out in the presence of an alcohol or nitrile which is immiscible or only partially miscible with water as diluent.

3. Process according to Claims 1 or 2, **characterized in that** the diluent is chosen from the series n-butanol, amyl alcohol, n-propionitrile or butyronitrile.

4. Process according to one or more of Claims 1 to 3, **characterized in that** the mixture of CCMP/CMP and the corresponding hydrochlorides is obtained by chlorinating CMP in an organic solvent with free-radical initiator at the boil to a conversion of about 40% and then distilling the organic solvent under reduced pressure.

5. Process according to Claim 4, **characterized in that** the mixture of CCMP/CMP and the corresponding hydrochlorides following distillation of the solvent comprises 5 - 15% residue solvent, 30 - 50% CMP and 25- 45% CCMP with the corresponding hydrochlorides.

6. Process according to one of Claims 1 to 5, **characterized in that** the compounds of the formula (II) used are the compound of the formula (IIa) or the compound of the formula (IIb)

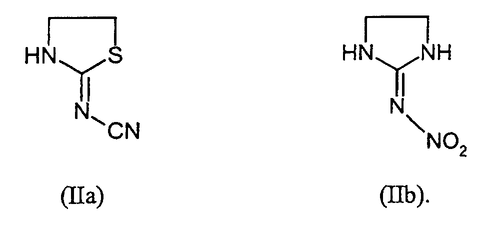

## Revendications

1. Procédé de préparation de composés de la formule (I) : 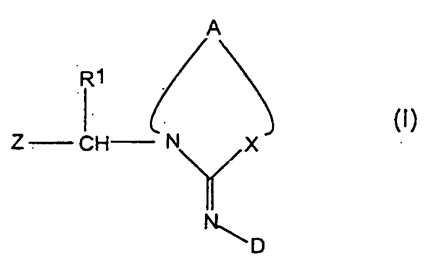 dans laquelle :
R¹ représente l'atome d'hydrogène ;
A représente un radical éthylène, qui peut être substitué par un radical alcoyle, ou un radical triméthylène, qui peut être substitué par un radical alcoyle ;
D représente le radical nitro ou cyano ;
X représente l'atome d'oxygène ou l'atome de soufre ou les radicaux 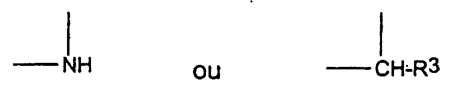
où
R³ représente l'atome d'hydrogène ou un radical alcoyle, et
Z représente le radical 2-chloropyrid-5-yle,
**caractérisé en ce que** l'on fait réagir des composés de la formule (II) : 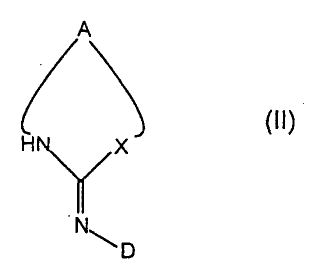 dans laquelle A, D et X ont les significations indiquées ci-dessus,
avec une base et en présence d'un agent de dilution et **en ce que** le mélange réactionnel est ensuite mis à réagir avec le mélange CCMP/CMP (2-chloro-5-chlorométhylpyridine/2-chloro-5-méthylpyridine) et les chlorhydrates correspondants.

2. Procédé suivant la revendication 1, **caractérisé en ce que** la réaction est réalisée en présence d'un alcool ou d'un nitrile non ou seulement partiellement miscible à l'eau comme agent de dilution.

3. Procédé suivant la revendication 1 ou 2, **caractérisé en ce que** l'agent de dilution est choisi parmi la série du n-butanol, de l'amylalcool, du n-propionitrile ou du butyronitrile.

4. Procédé suivant l'une ou plusieurs des revendications 1 à 3, **caractérisé en ce que** le mélange de CCMP/CMP et des chlorhydrates correspondants est obtenus **en ce que** l'on chlore le CMP dans un solvant organique avec un initiateur radicalaire au reflux, jusqu'à une transformation d'environ 40% et ensuite, le solvant organique est distillé sous vide.

5. Procédé suivant la revendication 4, **caractérisé en ce que** le mélange de CCMP/CMP et des chlorhydrates correspondants contient après la distillation du solvant, 5-15% de solvant résiduel, 30-50% de CMP et 25-45% de CCMP avec les chlorhydrates correspondants.

6. Procédé suivant l'une ou plusieurs des revendications 1 à 5, **caractérisé en ce que** l'on met en oeuvre comme composés de la formule (II), le composé de la formule (IIa) ou le composé de la formule (IIb) :
